# EUROPÄISCHE PATENTSCHRIFT

(11) **EP 0 476 335 B1**
(45) Veröffentlichungstag und Bekanntmachung des Hinweises auf die Patenterteilung: **31.07.1996**
(21) Anmeldenummer: 91113967.3
(22) Anmeldetag: 21.08.1991
(51) Int. Cl.: A61L 2/18, A61L 2/02

(54) **Medizinisches Desinfektionsgerät**
Device for medical disinfection
Appareil pour la désinfection médicale

(30) Priorität: 13.09.1990 DE 4029088
(43) Veröffentlichungstag der Anmeldung: 25.03.1992
(73) Patentinhaber: Fresenius AG, D-61350 Bad Homburg (DE)
(72) Erfinder: Kirschner, Ulrich, Dr. Dipl.-Chem., W-6082 Mörfelden-Walldorf (DE); Jethon, Frank, W-6380 Bad Homburg (DE)
(74) Vertreter: Fuchs, Luderschmidt & Partner Patentanwälte

(56) Entgegenhaltungen:
- EP-A- 0 050 864
- EP-A- 0 097 863
- EP-A- 0 312 104
- AT-A- 313 478

## Beschreibung

Die Erfindung betrifft ein medizinisches Desinfektionsgerät mit einer Desinfektionskammer, die über eine Wasserzulaufleitung mit einer Wasserquelle verbunden und eine Abwasserleitung aufweist, wobei in die Wasserzulaufleitung eine Desinfektionsmittelleitung mündet, die an ihrem anderen Ende mit einem ein Desinfektionsmittel aufweisenden Desinfektionsmittelbehälter verbunden ist, und mit einer in die Desinfektionsmittelleitung eingeschalteten Pumpe zur Förderung des Desinfektionsmittels.

Geräte der eingangs erwähnten Art werden üblicherweise im Bereich der Medizin zur Sterilisierung und/oder Desinfektion von Operationsgegenständen, Endoskopen, Spritzen udgl. eingesetzt, die nicht mit Heißluft und/oder Heißdampf bei überhöhten Temperaturen sterilisiert und/oder desinfiziert werden können. Zu diesem Zweck wird ein flüssiges Desinfektionsmittel, üblicherweise in Form eines Desinfektionsmittelkonzentrats, das mit Frischwasser vermischt wird, einer Desinfektionskammer zugeführt, in der die zu desinfizierenden Teile vorgelegt sind. Dort wird dann über einen vorbestimmten Zeitraum die Desinfektion durchgeführt, woraufhin nach einem bestimmten Programm das gebrauchte Desinfektionsmittel über den Ablauf ausgeschieden wird. Hieran schließt sich ein Spülvorgang zur Entfernung der Desinfektionsmittelreste an, woraufhin die desinfizierten Teile ggfls. unter Trocknen wieder aus der Kammer entnommen werden können.

Obwohl das zugeführte Frischwasser üblicherweise keine Keime aufweist und nahezu die gesamte Leitung mit Desinfektionsmittel gespült worden ist, treten immer noch Probleme mit Keimen, insbesondere Pseudomonaden (Pseudomonas aeruginosa) auf, die nicht aus dem Zuführungsbereich, zu beseitigen sind. Dies kann zu einer erneuten Verkeimung der bereits desinfizierten Geräte führen mit der Folge, daß solche kontaminierten Gerätschaften eine Gefahr für den zu behandelnden Patienten darstellen können.

Der Erfindung liegt daher die Aufgabe zugrunde, ein solches Desinfektionsgerät zur Verfügung zu stellen, das eine Kontamination nach dem Desinfizieren während des Freispülens mit Wasser sicher ausschließt.

Die Lösung der Aufgabe erfolgt dadurch, daß stromab des Mischpunkts von Frischwasser und Desinfektionsmittel in die Wasserzulaufleitung ein Sterilfilter mit einem Wasserfluß von mehr als 30 ml/hm²mmHg eingeschaltet ist.

Die erfindungsgemäße Vorrichtung weist nunmehr den Vorteil auf, daß das vor der Desinfektionskammer angeordnete Sterilfilter ein Einschleppen von Keimen in die Desinfektionskammer sicher verhindert. Dabei bleibt der zwischen dem Mischpunkt und dem Sterilfilter vorgesehene Leitungsabschnitt sowie der sich an das Sterilfilter bis zur Desinfektionskammer sich anschließende Leitungsabschnitt der Desinfektionsmittelzuleitung bis zur Zuführung von Frischwasser nach der Durchführung der Desinfektion steril. Da es zu einer Ablösung von Keimen aus dem Leitungsbereich stromauf des Mischpunkts kommen kann, werden diese Keime nur bis zur Membran des Sterilfilters transportiert und dort aufgrund der sehr viel geringeren Porengröße der Membran zurückgehalten, so daß sie nicht in den sterilen Bereich stromab des Sterilfilters gelangen können.

Insofern bleiben sämtliche mit flüssigem Desinfektionsmittel in der Desinfektionskammer behandelten Geräte auch nach dem Spülen mit sterilem Frischwasser, wie es ausgangs des Sterilfilters erhalten wird, sicher steril.

Als Sterilfilter wird vorteilhafterweise ein Membranfilter eingesetzt, der einen hohen Wasserfluß zuläßt, beispielsweise mehr als 30 ml/hm²mmHg. Besonders vorteilhaft werden High-Flux-Industriefilter eingesetzt, wie sie bei der üblichen Hämodialyse für die Sterilfiltration zum Einsatz kommen. Derartige Filter sind aufgrund ihrer Materialeigenschaften hydrophil und bestehen beispielsweise aus Polysulfon, das mit Hilfe von Polyvinylpyrrolidon hydrophilisiert wurde, Celluloseacetat, Polyacrylnitril udgl. Sie haben üblicherweise eine Membranoberfläche zwischen 1 und 3 m² und liegen gewöhnlich in Form eines Hohlfaserfilters vor, der ca. 9.000-10.000 Hohlfasern in einem im wesentlichen zylindrischen Gehäuse enthält. Derartige Hohlfasermembrane haben einen Innendurchmesser von etwa 0,2 mm, eine Wandstärke von ca. 20-30 µm und eine mittlere Porengröße unterhalb 0,5 µm, insbesondere unterhalb 0,1 µm. Besonders vorteilhaft wird ein hydrophilisierter und für Industriezwecke modifizierter Polysulfonfilter der Anmelderin mit der Bezeichnung F 60 oder F 80 in Form eines Hohlfaserfilters eingesetzt. Dabei ist das zylindrische Gehäuse, das diese Hohlfasern aufweist, an seinen Enden mit einer Vergußschicht verschlossen, in die die Hohlfasern eingebettet sind. Eine solche Vergußschicht kann beispielsweise aus Polyurethan bestehen, das in situ ausgehärtet ist, wobei anschließend durch ein teilweise erfolgendes Abschneiden der Enden die Faseröffnungen wieder geöffnet werden. Somit kann das wässrige Desinfektionsmedium stirnseitig in die Hohlräume (Lumina) der Hohlfasern eingeführt und durch die Poren der Hohlfasern in den Zwischenraum zwischen den Fasern in das Gehäuse und von dort durch eine Auslaßöffnung der Desinfektionskammer zugeführt werden.

Weitere Merkmale, Vorteile und Ausführungsformen der Erfindung sind aus der nachfolgenden Beschreibung eines Ausführungsbeispiels ersichtlich.

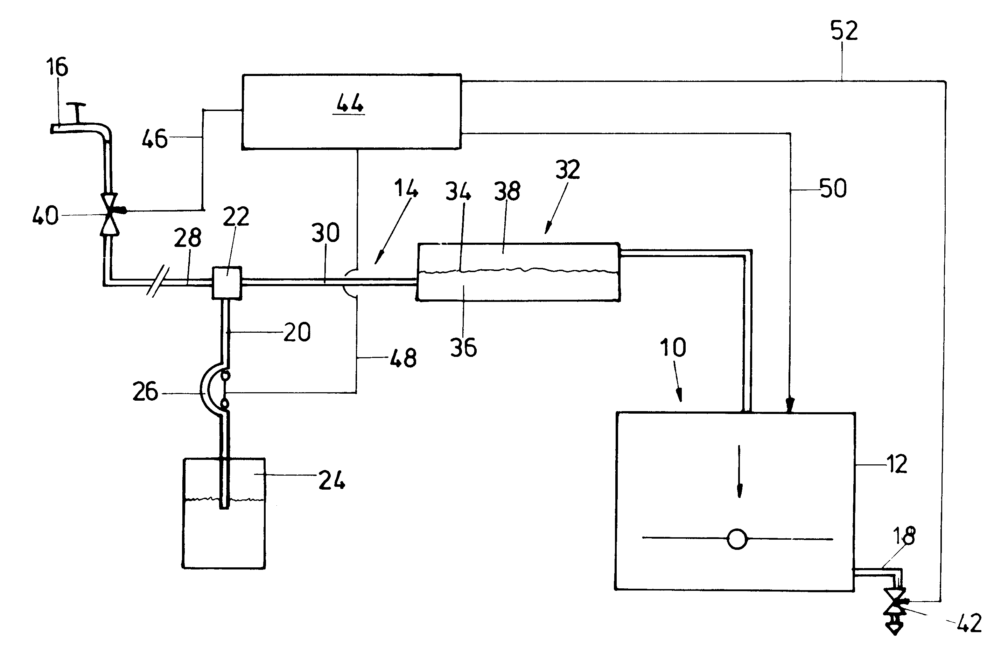

Die Figur zeigt eine schematische Skizze eines medizinischen Desinfektionsgeräts der Erfindung.

In der Figur ist ein Desinfektionsgerät 10 gezeigt, das eine Desinfektionskammer 12 aufweist, die mit üblichen, nicht gezeigten Behandlungseinrichtungen (Sprüharmen, Umwälzmitteln udgl.) ausgerüstet ist. Diese Desinfektionskammer nimmt die zu desinfizierenden Gegenstände auf.

Die Desinfektionskammer 12 ist über eine Wasserzulaufleitung 14 mit einer Frischwasserquelle 16 verbunden und weist andererseits eine Abwasserleitung 18 auf.

Weiterhin mündet in die Wasserzulaufleitung eine Desinfektionsmittelleitung 20 unter Bildung eines Mischpunktes 22. Das andere Ende der Desinfektionsmittelleitung 20 ist mit einem Desinfektionsmittelbehälter 24 verbunden, der das Desinfektionsmittel in Form eines Konzentrats aufweist. Weiterhin ist in die Desinfektionsmittelleitung 20 eine Pumpe 26 eingeschaltet, die das Desinfektionsmittelkonzentrat in Betrieb zum Mischpunkt 22 in einer vorbestimmten Menge fördert, im ausgeschalteten Zustand jedoch die Desinfektionsmittelleitung sperrt.

Der Mischpunkt 22 teilt dabei die Wasserzulaufleitung 14 in einen Leitungsabschnitt 28, der nur durch Frischwasser beaufschlagt wird, und einen zweiten Leitungsabschnitt 30 auf, der mit fertiger Desinfektionsmittellösung durchspült wird.

In den letztgenannten zweiten Leitungsabschnitt ist ein Sterilfilter 32 eingeschaltet, dessen Innenraum durch eine Membran 34 in eine erste Filterkammer 36 und eine zweite Filterkammer 38 getrennt ist. Dabei sind die erste Filterkammer 36 mit dem zugehenden Teil des zweiten Leitungsabschnitts 30 und die zweite Filterkammer 38 mit dem abgehenden Teil des zweiten Leitungsabschnitts 30 verbunden.

Schließlich weisen der erste Leitungsabschnitt 28 ein Wasserzulaufventil 40 und die Abwasserleitung ein Ablaufventil 42 auf. Schließlich ist ein Steuergerät 44 vorgesehen, das über Leitungen 46-52 mit dem Wasserzulaufventil 40, der Pumpe 26, der Desinfektionskammer 12 bzw. dem Abwasserventil 42 verbunden ist.

### Das Desinfektionsgerät 10 wird folgendermaßen betrieben:

Zur Desinfektion wird die mit den zu desinfizierenden Gerätschaften versehene Desinfektionskammer 12 mit frischer Desinfektionsmittellösung beaufschlagt. Hierzu wird das Wasserzulaufventil 40 geöffnet und die Pumpe 26 zur Zuführung von flüssigem Desinfektionsmittelkonzentrats (beispielsweise das Produkt der Anmelderin mit der Bezeichnung "Ultrascope", "Puristeril", "Sporcid M") aktiviert. Da sowohl Frischwasser als auch das Konzentrat in vorbestimmten Mengen dem Mischpunkt 22 zugeführt werden, ist die Zusammensetzung und Menge der Desinfektionsmittellösung bekannt. Das fertige Desinfektionsmittel wird durch den zweiten Leitungsabschnitt 30 der Wasserzuführungsleitung 14 und somit durch das Sterilfilter hindurch gepumpt und der Desinfektionskammer 12 zugeführt. Nach Erreichen eines bestimmten Füllstandes in der Desinfektionskammer 12 werden sowohl das Wasserzulaufventil 40 als auch die Pumpe 26 desaktiviert mit der Folge, daß im zweiten Leitungsabschnitt das flüssige Desinfektionsmittel stehenbleibt und somit diesen gesamten Abschnitt einschließlich des Sterilfilters 32 wirksam desinfiziert. Hierauf wird die Desinfektionskammer 12 in Betrieb gesetzt, wobei das Abwasserventil 42 desaktiviert bleibt.

Nachdem das Desinfektionsprogramm abgelaufen ist, wird das Abwasserventil 42 aktiviert, so daß das flüssige Desinfektionsmittel aus der Desinfektionskammer 12 entfernt wird.

Hierauf setzt der Spülvorgang unter Öffnung des Wasserzulaufventils 40 mit Frischwasser ein, mit der Folge, daß die gesamte Wasserzulaufleitung 14 und die Desinfektionskammer 12 mit Frischwasser in einem bestimmten Programm freigespült werden. Eventuell in der Wasserzulaufleitung 14 befindliche Keime werden sicher durch das Sterilfilter 32 zurückgehalten, so daß das Desinfektionsgerät 10 bis zum Öffnen steril bleibt.

Nach dem Spülen mit Frischwasser erfolgen nach dem üblichen Programm das Trocknen und die Entnahme der sterilisierten Geräte.

## Patentansprüche

1. Medizinisches Desinfektionsgerät (10) mit einer Desinfektionskammer (12), die über eine Wasserzulaufleitung (14) mit einer Frischwasserquelle (16) verbunden ist und eine Abwasserleitung (18) aufweist, wobei in die Wasserzulaufleitung eine Desinfektionsmittelleitung (20) mündet, die an ihrem anderen Ende mit einem ein Desinfektionsmittel aufweisenden Desinfektionsmittelbehälter (24) verbunden ist, und mit einer in die Desinfektionsmittelleitung (20) eingeschalteten Pumpe (26) zur Förderung des Desinfektionsmittels, dadurch gekennzeichnet, daß stromab des Mischpunkts (22) von Frischwasser und Desinfektionsmittel in die Wasserzulaufleitung (14) ein Sterilfilter (32) mit einem Wasserfluß von mehr als 30 ml/hm²mmHg eingeschaltet ist.

2. Gerät nach Anspruch 1, dadurch gekennzeichnet, daß der Sterilfilter (32) aus einer Vielzahl von mikroporösen Hohlfasern besteht, wobei die Membran eine mittlere Porengröße unterhalb 0,5 µm aufweist.

## Claims

1. A medical disinfection apparatus (10) comprising a disinfection chamber (12), which is connected with a fresh water source (16) by means of a water inlet (14) and which has a water outlet (18), further comprising a disinfectant conduit (20) which runs into the water inlet and is connected at its other end with a disinfectant reservoir (24) containing a disinfectant, further comprising a pump (26) coupled in the disinfectant conduit (20) for pumping the disinfectant, characterized in that a sterile filter (32) with a water flux of more than 30 ml/hm²mmHg is provided downstream of the mixing point (22) of the fresh water and disinfectant.

2. The apparatus according to claim 1, characterized in that the sterile filter (32) comprises a plurality of microporous hollow fibers, which form a membrane having an average pore size of less than 0.5 µm.

## Revendications

1. Appareil médical de désinfection (10) comportant une chambre de désinfection (12), qui est reliée par l'intermédiaire d'une canalisation d'arrivée d'eau (14) à une source d'eau fraîche (16), et comporte une canalisation (18) d'évacuation des eaux usées, et dans lequel dans la canalisation d'arrivée d'eau débouche une canalisation (20) pour un agent désinfectant, qui est reliée, par son autre extrémité, à un récipient (24) pour l'agent désinfectant, qui contient un agent désinfectant, et comportant une pompe (26) montée dans la canalisation (20) pour l'agent désinfectant de manière à entraîner l'agent désinfectant, caractérisé en ce qu'en aval du point de mélange (22) de l'eau fraîche et de l'agent désinfectant est monté, dans la canalisation d'arrivée d'eau (14), un filtre stérile (32) avec un débit d'eau supérieur à 30 ml/hm²mmHg.

2. Appareil selon la revendication 1, caractérisé en ce que le filtre stérile (32) est constitué par une multiplicité de fibres creuses microporeuses, la membrane possédant une taille moyenne de pores inférieure à 0,5 µm.
